# EUROPÄISCHE PATENTSCHRIFT

(11) **EP 3 708 051 B1**
(45) Veröffentlichungstag und Bekanntmachung des Hinweises auf die Patenterteilung: **26.07.2023**
(21) Anmeldenummer: 19163232.2
(22) Anmeldetag: 15.03.2019
(51) Int. Cl.: A47K 5/12, G08B 21/24, G09B 19/00

(54) **FLÜSSIGKEITSSPENDER UND VERFAHREN ZUM ANLEITEN EINER KÖRPERREINIGUNGSPROZEDUR**
FLUID DISPENSER AND METHOD FOR GUIDING A BODY CLEANING PROCEDURE
DISTRIBUTEUR DE LIQUIDE ET PROCÉDÉ DE GUIDAGE D'UNE PROCÉDURE DE NETTOYAGE DU CORPS

(43) Veröffentlichungstag der Anmeldung: 16.09.2020
(73) Patentinhaber: OP-Hygiene IP GmbH, 4704 Niederbipp (CH); Ophardt Hygiene-Technik GmbH + Co. KG, 47661 Issum (DE)
(72) Erfinder: Becker, Karsten, 17509 Hanshagen (DE); Knaack, Dennis, 48356 Nordwalde (DE); Klar, Kristin, 49545 Tecklenburg (DE); Ophardt, Heiner, 4422 Arisdorf (CH); Ruhnau, Anna, 46483 Wesel (DE); Steltenkamp, Siegfried, 53229 Bonn-Niederholtorf (DE); Harmes, Martin, 47929 Grefrath (DE); Stressler, Patrick, 40668 Meerbusch (DE)
(74) Vertreter: Grünecker Patent- und Rechtsanwälte PartG mbB

(56) Entgegenhaltungen:
- US-A1- 2010 282 772
- US-A1- 2014 375 457
- US-A1- 2017 086 625

## Beschreibung

Die vorliegende Erfindung betrifft Flüssigkeitsspender für Hygieneflüssigkeiten und zugehörige Verfahren für Körperreinigungsprozeduren, insbesondere für Handreinigungsprozeduren.

Aus dem Stand der Technik sind Hygienespender bekannt, die, ausgelöst durch einen Betätigungs- oder Bewegungsmelder, anfangs kurze visuelle Botschaften an den Bediener ausgeben. Die Art der visuellen Botschaften ist hierbei unterhaltsamer Natur.

Weiterhin ist aus der US 2017/0086625 A1 ein Flüssigkeitsspender mit einem Display bekannt, welches dem Benutzer visuelle Waschhinweise gibt.

Die US 2014/0375457 A1 offenbart ein Handhygienemodul mit einer Anleitungsoberfläche mit wandernden Abbildungen zu vorzunehmenden Waschschritten.

Die US 2010/0282772 A1 offenbart einen automatischen Flüssigkeitsspender mit einer Displayanzeige, welche Waschanweisungen wiedergibt.

Es hat sich jedoch gezeigt, dass derartige Hygienespender nur unzureichende Waschergebnisse erzielen, insbesondere bei Bedienern mit nicht voll ausgebildeten kognitiven Fähigkeiten, wie beispielsweise bei Kindern, älteren Menschen oder Demenzkranken.

Der vorliegenden Erfindung liegt die Aufgabe zugrunde, einen Flüssigkeitsspender sowie ein Verfahren bereitzustellen, welches im Vergleich zu bestehenden Systemen beim Benutzer ein besseres Reinigungsergebnis fördert.

Die Erfindung löst die Aufgabe mit einem Flüssigkeitsspender gemäß des unabhängigen Anspruchs 1.

Hierbei wird ein Flüssigkeitsspender zum Anleiten einer Körperreinigungsprozedur, insbesondere einer Handreinigungsprozedur, vorgeschlagen, welcher eine betätigbare Ausgabeeinheit für Hygieneflüssigkeit, und eine optische Anzeigeeinheit, welche mit der Ausgabeeinheit in Verbindung steht, aufweist, wobei der Flüssigkeitsspender dazu eingerichtet ist, dass die optische Anzeigeeinheit, nach einer Betätigung der Ausgabeeinheit, einem Bediener visuelle Signale über eine hinterlegte Zeitdauer einer empfohlenen Körperreinigungsprozedur anzeigt.

Eine visuelle Signalanzeige für die Zeitdauer einer empfohlenen Körperreinigungsprozedur gibt dem Bediener ein anhaltendes visuelles Feedback über die Dauer der Körperreinigungsprozedur und fördert hierdurch ein ausreichend langes Reinigen des Körpers oder Körperteile gemäß der hinterlegten Zeitdauer. Die Zeitdauer kann beispielsweise auf einem Datenspeicher im Flüssigkeitsspender hinterlegt sein und je nach Körperreinigungsprozedur beispielsweise die zeitliche Empfehlung der Weltgesundheitsorganisation (WHO) wiedergeben. In möglichen Ausführungsformen liegt die hinterlegte Zeitdauer im Bereich von ca. 10 Sekunden bis ca. 3 Minuten. In einer möglichen Ausführungsform wäre die hinterlegte Zeitdauer für eine Handwaschprozedur ca. 30 Sekunden (alltägliche Handwaschprozedur) oder ca. 3 Minuten (chirurgische Desinfektion).

Die optische Anzeigeeinheit ist dazu eingerichtet, gleichzeitig unterschiedliche visuelle Signale zu parallel ablaufenden Körperreinigungsprozeduren mehrerer Bediener anzuzeigen.

Dies ermöglicht es, dass der Flüssigkeitsspender gleichzeitig von mehreren Bedienern genutzt werden kann, wobei trotzdem jeder Bediener erfindungsgemäße Anweisungen zu empfohlenen Körperreinigungsprozeduren erhält.

Zudem weist die optische Anzeigeeinheit mehrere optische Fortschrittsanzeigen für Körperreinigungsprozeduren in Form von mehreren nebeneinanderliegenden optischen Anzeigebahnen auf. In einer optionalen Ausführungsform können die optischen Anzeigebahnen sektionierte Lichtleisten aufweisen.

Durch den Einsatz einer optischen Fortschrittsanzeige kann der Bediener zu jeder Zeit auf einem Blick erkennen, an welcher Stelle im Körperreinigungsprozess er sich gerade befindet, was die Motivation fördert, die empfohlene Körperreinigungsprozedur bis zum Ende mitzumachen, was wiederum das Reinigungsergebnis verbessert.

Bei einem Einsatz von mehreren optischen Fortschrittsanzeigen kann jeder Bediener an seiner zugehörigen Fortschrittsanzeige seinen zugehörigen Reinigungsprozessfortschritt ablesen.

Nebeneinanderliegende optische Anzeigebahnen können gerade bei Bedienern mit nicht voll ausgebildeten kognitiven Fähigkeiten die Benutzungsmotivation erhöhen, da das Gemeinschaftserlebnis gefördert wird, einschließlich möglicher kompetitiver Aspekte zwischen den Bedienern, was unter anderem die Benutzungsmotivation und somit das Reinigungsergebnis fördert.

In einer möglichen Ausführungsform kann der Flüssigkeitsspender dazu eingerichtet sein, dass sich die visuellen Signale entsprechend einer hinterlegten zeitlichen Abfolge derart verändern, dass es einem Bediener möglich ist, diese Veränderungen unterschiedlichen Handlungsschritten einer empfohlenen Körperreinigungsprozedur zuzuordnen.

Dadurch kann der Bediener während der Waschprozedur mit zusätzlichen Informationen versorgt werden, zu welchem Zeitpunkt und über welche Dauer in welcher Art die Körperreinigung durchgeführt werden soll. Hierdurch können zusätzliche Verbesserungen beim Körperreinigungsergebnis erzielt werden und der Bediener kann in Echtzeit durch die unterschiedlichen Schritte einer empfohlenen Körperreinigungsprozedur geführt werden.

Gerade bei Bedienern mit nicht voll ausgebildeten kognitiven Fähigkeiten und/oder eingeschränktem Gehörsinn, kann durch die erfindungsgemäße Kombination von prozedurbegleitendem visuellen Feedback bei gleichzeitiger dargebotener visueller Information über den jeweils vorzunehmenden Reinigungsschritt, ein verbessertes Körperreinigungsergebnis erzielt werden.

In einer möglichen Ausführungsform können die optischen Fortschrittsanzeigen jeweils dazu eingerichtet sein, die visuellen Signale von einem Startpositionsfeld der optischen Fortschrittsanzeige hin zu einem Endpositionsfeld der optischen Fortschrittsanzeige laufen zu lassen, insbesondere intermittierend und/oder translatorisch laufen zu lassen.

Durch eine solche Ausführung findet sich der Bediener leicht zurecht, an welcher Stelle der Körperreinigungsprozedur er sich gerade befindet. Anfang und Ende der Körperreinigungsprozedur sind sofort ersichtlich. Dies fördert die Motivation die Körperreinigung über die empfohlene Gesamtdauer zu vollziehen.

Ein optionales intermittierendes und/oder translatorisches Laufenlassen der visuellen Signale, beispielsweise in Form eines sich bewegenden punktuellen Signals, gibt dabei sehr einfach und informativ den Jetzt-Zustand der empfohlenen Reinigungsprozedur wieder.

In einer empfohlenen möglichen Ausführungsform kann die optische Anzeigeeinheit, insbesondere die optischen Fortschrittsanzeigen, dazu eingerichtet sein, die visuellen Signale positionsabhängig inhaltlich zu verändern, insbesondere positionsabhängig in unterschiedlichen Farben anzuzeigen.

Dies ermöglicht in effizienter Weise die Information über den zeitlichen Ist-Zustand der Körperreinigungsprozedur mit weiteren Informationen zu den jeweils gerade empfohlenen Handlungsschritten der Prozedur zu kombinieren. Eine mögliche Realisierung durch unterschiedliche Farben ermöglicht die Implementierung der gewünschten Zusatzinformationen zu den jeweiligen Handlungsschritten in einfacher und verlässlicher Weise. Hierbei kann der Bediener beispielsweise mit jeder Farbe einen oder mehrere definierte Handlungsschritte assoziieren, beispielsweise durch den Einsatz von zusätzlichen Informationsquellen, welche besagte Assoziationen beim Bediener herstellen.

Günstigerweise können in einer möglichen Ausführungsform die optischen Fortschrittsanzeigen jeweils an ihrem Endpositionsfeld ein im Vergleich zu den vorangegangenen Positionen der optischen Fortschrittsanzeige visuell hervorgehobenes Signal ausgeben, und/oder können mehrere optische Fortschrittsanzeigen ein gemeinsames Endpositionsfeld aufweisen.

Eine derartige Ausführung des Endpositionsfeldes erlaubt es bei Erreichen der Endposition, also bei Beendigung der empfohlenen Reinigungsprozedur, ein besonderes visuelles Signal anzuzeigen, was dem Bediener neben der Beendigung auch einen Erfolg signalisiert und so zur Benutzungsmotivation und dem Reinigungserfolg beiträgt.

Eine optionale Ausführung von einem gemeinsamen Endpositionsfeld für mehrere Fortschrittsanzeigen erlaubt es das Endpositionsfeld größer und visuell auffälliger zu gestalten und den Spender gleichzeitig effizient auszuführen. Zudem wird gerade bei Bedienern mit nicht voll ausgebildeten kognitiven Fähigkeiten, wie beispielsweise Kindern, das Gemeinschaftsgefühl und somit die Benutzungsmotivation und das Reinigungsergebnis gefördert.

In einer vorgeschlagenen Ausführungsform kann der Flüssigkeitsspender einen Empfänger, insbesondere einen Funkempfänger, aufweisen, welcher ein externes Türöffnungssignal empfangen kann, und der Flüssigkeitsspender kann dazu eingerichtet sein, bei einem Empfang eines Türöffnungssignals mindestens ein visuelles Signal auf der optischen Anzeigeeinheit anzuzeigen.

Eine solche Ausführung ermöglicht es, einen Bediener bereits beim Betreten eines Waschraums über visuelle Signale des Spenders anzusprechen. Gleichzeitig kann ein solches visuelles Signal auf der Anzeigeeinheit bereits ein erstes Einstiegssignal für die empfohlene Körperreinigungsprozedur darstellen und den Bediener beispielsweise anleiten, sich noch vor Betätigen der Ausgabeeinheit die Hände zu befeuchten.

Eine derartige Ausführung erhöht den Benutzungsgrad des Spenders und fördert das Reinigungsergebnis.

In einer möglichen Variante der Erfindung kann der Flüssigkeitsspender eine berührungslose Überwachungssensorik aufweisen, welche mit der optischen Anzeigeeinheit verbunden ist und welche dazu eingerichtet ist, während einer Körperreinigungsprozedur die Bewegungen und/oder Positionen eines Bedieners zu detektieren und basierend auf hinterlegten Bewegungs- und/oder Positionsmustern zu bewerten, und die optische Anzeigeeinheit kann dazu eingerichtet sein, die visuellen Signale bei einer Körperreinigungsprozedur in Abhängigkeit von der Bewertung der Überwachungssensorik anzuzeigen.

Hierdurch wird eine mehrfache berührungslose Interaktion zwischen einem Bediener und dem Flüssigkeitsspender ermöglicht, wobei der Flüssigkeitsspender über die gesamte Dauer der Körperreinigungsprozedur eine Rückmeldung vom Bediener über die Umsetzung der Prozedur erhalten kann, und die visuelle Signalanzeige entsprechend anpassen kann. So kann ein solcher Spender beispielsweise registrieren, dass ein Bediener die Prozedur vorzeitig abgebrochen hat und die Signalanzeige entsprechend stoppen und beispielsweise auch ein Beendigungssignal oder Endpositionsfeld nicht anzeigen, welches eine Voraussetzung für nachgeschaltete Aktionen sein kann, welche dann nicht ausgelöst werden.

In einer möglichen speziellen Ausführungsform kann die berührungslose Überwachungssensorik mindestens einen Bewegungsmelder und/oder mindestens eine Kamera aufweisen.

Mit einem Bewegungsmelder kann in einfacher Weise überwacht werden, ob ein Bediener während der Dauer der empfohlenen Körperreinigungsprozedur in der Nähe des Flüssigkeitsspenders agiert.

Durch den Einsatz einer Kamera, beispielsweise einer Videokamera, können Details zu den vom Bediener ausgeführten Bewegungen bis hin zu typischen Waschbewegungen überwacht werden.

In einer weiteren möglichen Ausführungsform kann der Flüssigkeitsspender einen Sender, insbesondere einen Funksender, aufweisen, welcher ein Signal an einen externen Empfänger, insbesondere an ein Durchgangsöffnungs- und Schließsystem, senden kann, und der Flüssigkeitsspender dazu eingerichtet ist, ein erfolgtes vollständiges Durchlaufen einer Körperreinigungsprozedur an den externen Empfänger, insbesondere an das Durchgangsöffnungs- und Schließsystem, zu melden.

Hierdurch können Systeme realisiert werden, in denen ein vollständiges Durchlaufen einer Körperreinigungsprozedur Voraussetzung für nachgeschaltete Aktionen von externen Empfangsgeräten ist. So kann in einer möglichen Ausführungsform ein Zugangskontrollsystem geschaffen werden, wobei der Zugang zu einem verschlossenen hygienesensitiven Bereich durch ein Durchgangsöffnungs- und Schließsystem erst nach vorherigem Empfang eines Signals über das erfolgte vollständige Durchlaufen einer Körperreinigungsprozedur freigegeben wird.

Ebenfalls möglich ist in einer Ausführungsform ein Körperreinigungssystem, insbesondere Handreinigungssystem, gekennzeichnet durch einen zuvor genannten Flüssigkeitsspender, insbesondere den Flüssigkeitsspender mit genannter Überwachungselektronik, und durch mindestens einen externen berührungslosen Bewegungsmelder, welcher im Umfeld des Flüssigkeitsspenders installierbar ist und mit dem Flüssigkeitsspender kommunizieren kann, wobei der Flüssigkeitsspender, insbesondere die Überwachungssensorik, dazu eingerichtet ist, aus den gewonnenen Bewegungs- und/oder Positionsdaten des Bedieners Rückschlüsse auf die Aktionen des Bedieners zu ziehen und diese zu bewerten, und die optische Anzeigeeinheit dazu eingerichtet ist, die visuellen Signale bei einer Körperreinigungsprozedur in Abhängigkeit von der Bewertung anzuzeigen.

Durch den Einsatz eines externen berührungslosen Bewegungsmelders kann der Flüssigkeitsspender noch situativer auf das Verhalten des Bedieners in einem weiteren Umfeld des Flüssigkeitsspenders reagieren und die visuelle Signalausgabe an die detektierten Verhaltensmuster anpassen. Dies kann den Reinigungserfolg weiter steigern.

Ebenfalls möglich ist in einer Ausführungsform ein Körperreinigungssystem, insbesondere Handreinigungssystem, gekennzeichnet durch einen zuvor genannten Flüssigkeitsspender, und eine visuelle Informationsquelle, insbesondere eine Körperreinigungsanleitung, beispielsweise eine Handreinigungsanleitung, welche unterschiedliche optische Merkmale, insbesondere unterschiedliche Farbkennzeichnungen, entsprechend einzelner Phasen einer Körperreinigungsprozedur enthält, und die optische Anzeigeeinheit des Flüssigkeitsspenders derart eingerichtet ist, dass die angezeigten visuellen Signale zu einer Körperreinigungsprozedur, je nach Zeitfortschritt, besagte phasenzugehörige optische Merkmale, insbesondere die entsprechenden Farbkennzeichnungen, beinhalten.

Durch die Implementierung der phasenabhängigen optischen Merkmale aus der visuellen Informationsquelle in die visuellen Signale der optischen Anzeigeeinheit des Flüssigkeitsspenders kann der Informationsgehalt des visuellen Anzeigesignals mit Informationen zu den einzelnen Reinigungsphasen bzw. Reinigungsschritten erweitert werden, ohne diese gesamte Information in die Anzeigeeinheit selbst implementieren zu müssen. So kann die Anzeigeeinheit vergleichsweise einfach gehalten werden. Auch kann durch eine Abänderung der visuellen Informationsquelle der Informationsgehalt der visuellen Anzeigesignale geändert werden, ohne die optische Anzeigeeinheit des Flüssigkeitsspenders selbst ändern zu müssen.

Die anfangs genannte Aufgabe wird weiterhin durch ein Verfahren gemäß Anspruch 12 gelöst.

Hierbei handelt es sich um ein Verfahren zum Anleiten einer Körperreinigungsprozedur, insbesondere einer Handreinigungsprozedur, umfassend ein Detektieren einer Betätigung einer Ausgabeeinheit eines Hygieneflüssigkeitsspenders durch einen Bediener, gekennzeichnet durch ein Anzeigen von visuellen Signalen über eine hinterlegte Zeitdauer einer empfohlenen Körperreinigungsprozedur auf einer optischen Anzeigeeinheit. Weiterhin ist das Verfahren dadurch gekennzeichnet, dass bei einer Betätigung der Ausgabeeinheit während einer bereits laufenden Signalanzeige einer Körperreinigungsprozedur auf einer Fortschrittsanzeige in Form einer Anzeigebahn der Anzeigeeinheit, gleichzeitig visuelle Signale für eine weitere Körperreinigungsprozedur eines weiteren Bedieners auf einer weiteren Fortschrittsanzeige in Form einer weiteren Anzeigebahn der optischen Anzeigeeinheit angezeigt werden.

Hierdurch können mehrere Bediener gleichzeitig visuell durch empfohlene Körperreinigungsprozeduren geführt werden.

Weitere Vorteile eines solchen Verfahrens ergeben sich aus den vorherigen Erläuterungen zu den Vorteilen der Merkmale des zugehörigen Anspruchs 1.

Die optische Ausgabeeinheit kann nach dem erfindungsgemäßen Verfahren in einer Ausführungsform als Teil des Flüssigkeitsspenders und in einer anderen Ausführungsform als separates Gerät zum Flüssigkeitsspender ausgeführt sein.

In einer weiteren möglichen Ausführungsform können sich beim Anzeigen die visuellen Signale entsprechend einer hinterlegten zeitlichen Abfolge derart verändern, dass es einem Bediener möglich ist, diese Veränderungen unterschiedlichen Handlungsschritten einer empfohlenen Körperreinigungsprozedur zuzuordnen.

Die Vorteile eines solchen Verfahrens ergeben sich aus den vorherigen Erläuterungen zu den Vorteilen der Merkmale des zugehörigen Anspruchs 2.

Eine mögliche Ausführungsform betrifft ein zuvor genanntes Verfahren, wobei dem Bediener während dem Anzeigen der sich verändernden visuellen Signale, zusätzliche unterschiedliche optische Merkmale, insbesondere unterschiedliche Farbkennzeichnungen, präsentiert werden, welche einzelnen Phasen einer empfohlenen Körperreinigungsprozedur entsprechen, und die visuellen Signale der optischen Anzeigeeinheit, je nach Zeitfortschritt, besagte phasenzugehörige optische Merkmale, insbesondere die entsprechenden Farbkennzeichnungen, enthalten.

Durch die Implementierung der phasenabhängigen optischen Merkmale in die visuellen Signale der optischen Anzeigeeinheit kann der Informationsgehalt des visuellen Anzeigesignals mit Informationen zu den einzelnen Reinigungsphasen bzw. Reinigungsschritten erweitert werden, ohne diese gesamte Information in die Anzeigeeinheit selbst implementieren zu müssen. So kann die Anzeigeeinheit vergleichsweise einfach gehalten werden. Auch kann durch eine Abänderung der visuellen Informationsquelle der Informationsgehalt der visuellen Anzeigesignale geändert werden, ohne die optische Anzeigeeinheit selbst ändern zu müssen.

Hygieneflüssigkeiten im Sinne der Erfindung umfassen in einer Ausführungsform Körperreinigungsflüssigkeiten, beispielsweise Flüssigseifen oder Desinfektionsmittel.

Visuelle Signale im Sinne der Erfindung umfassen in einer möglichen Ausführungsform auch ein visuelles Dauersignal.

Eine weitere möglichen Ausführungsform umfasst einen zuvor genannten Flüssigkeitsspender, wobei der Flüssigkeitsspender dazu eingerichtet ist, am Ende einer vollständig durchlaufenen Körperreinigungsprozedur ein akustisches Signal von einem integrierten Lautsprecher und/oder ein Duftsignal von einem integrierten Duftspender abzugeben.

Anhand der nachfolgend genannten Figuren werden mögliche Ausführungsformen der Erfindung näher erläutert. Besagte Ausführungsformen stellen dabei vorteilhafte Kombinationen mehrerer zuvor genannter Ausführungsformen dar. Gleiche Merkmale haben dabei gleiche Referenzzeichen.

Es zeigen
- Figur 1: eine perspektivische Frontansicht eines erfindungsgemäßen Flüssigkeitsspenders,
- Figur 2: eine Frontansicht des Flüssigkeitsspenders aus Figur 1,
- Figur 3: ein Installationsbeispiel für einen erfindungsgemäßen Flüssigkeitsspender zusammen mit einer visuellen Informationsquelle, welche zusammen ein erfindungsgemäßes Körperreinigungssystem bilden,
- Figur 4: unterschiedliche Anzeigezustände des Flüssigkeitsspenders aus Fig. 1 in einer linken Fortschrittsanzeige, wobei das jeweils aktive Signalfeld gestrichelt umrandet und die zeitliche Anzeigeabfolge durchnummeriert ist,
- Figur 5: Beispiel einer visuellen Informationsquelle für einen erfindungsgemäßen Flüssigkeitsspender,
- Figur 6: eine Frontansicht einer weiteren Ausführungsform eines erfindungsgemäßen Flüssigkeitsspenders bei der Anzeige von drei parallel laufenden Körperreinigungsprozeduren.

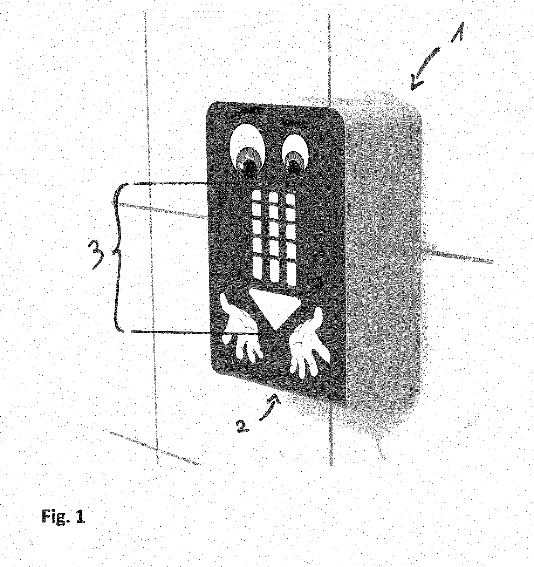

Figur 1 zeigt einen erfindungsgemäßen Flüssigkeitsspender 1, hier in einer wandhängenden, batteriebetriebenen Ausführung als Handseifenspender.

Der Flüssigkeitsspender 1 weist in dieser Ausführung eine betätigbare Ausgabeeinheit 2 zum portionierten Ausgeben von Hygieneflüssigkeit, hier von Flüssigseife auf. Die Ausgabeeinheit 2 umfasst hierbei eine elektrische Pumpeinheit (nicht dargestellt) mit einer Dosiereinheit (nicht dargestellt) sowie einen wechselbaren Hygieneflüssigkeitsbehälter (nicht dargestellt) und einen berührungslosen Körperteilsensor (nicht dargestellt), hier einen Handsensor ausgeführt als Infrarot-Sensor. Dosiereinheit und Handsensor sind hierbei an der Unterseite das Flüssigkeitsspenders 1 angebracht.

An der dem Raum zugewandten Frontseite des Flüssigkeitsspenders 1 ist eine elektrisch gesteuerte optische Anzeigeeinheit 3 angeordnet.

Die optische Anzeigeeinheit 3 ist in diesem Ausführungsbeispiel zentriert auf der Frontseite des Flüssigkeitsspenders 1 angeordnet und formt mit anderen auf der Frontseite angebrachten Illustrationen ein Teil eines Gesichts, um beispielsweise auf Kinder besonders ansprechend zu wirken.

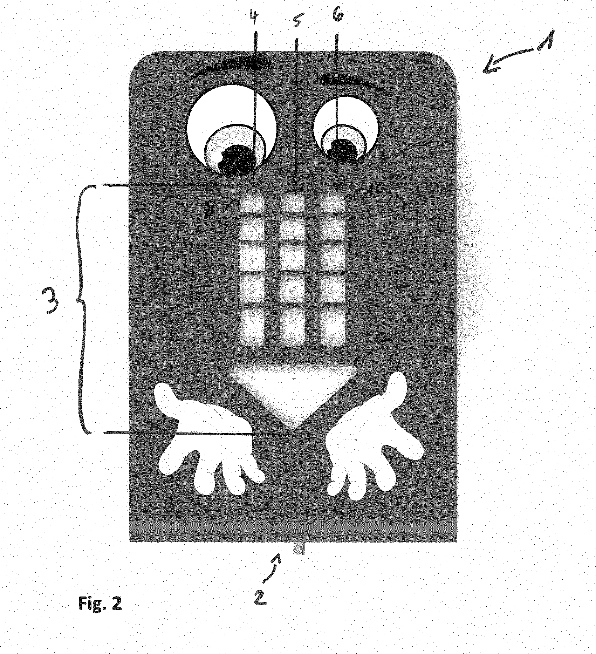

Wie in Figur 2 besser zu sehen ist, umfasst die optische Anzeigeneinheit 3 in diesem Ausführungsbeispiel drei vertikal und parallel zueinander angeordnete Fortschrittsanzeigen 4, 5, 6 gleicher Länge, welche hier in Form von in einzelne Anzeigefelder sektionierte LED-Lichtleisten realisiert sind. Die Sektionierung ist hier bei allen Fortschrittsanzeigen 4, 5, 6 gleich. Jede der drei Fortschrittsanzeigen 4, 5, 6 endet unten in einem gemeinsamen, hier großen und dreieckigen Endpositionsfeld 7.

Die drei Fortschrittsanzeigen 4, 5, 6 teilen sich somit hier ein gemeinsames Endpositionsfeld 7.

In jeder Fortschrittsanzeige 4, 5, 6 kann vom obersten Startpositionsfeld 8, 9, 10 ein visuelles Signal, hier in Form eines ortsabhängig die Farbe wechselnden, kontinuierlich blinkenden Lichtsignals, über Anzeigezwischenfelder nach unten in Richtung des großen dreieckigen Endpositionsfeldes 7 wandern.

Die Fortschrittsanzeigen 4, 5, 6 sind in dieser Ausführungsform jeweils so ausgeführt, dass die ersten oberen zwei Anzeigefelder rot blinken können, wobei das oberste Anzeigefeld 8, 9, 10 das Startpositionsfeld ist. Die anschließend nach unten nachfolgenden drei Anzeigefelder können gelb blinken und das große dreieckige Endpositionsfeld 7, das hier an eine lachende Mundöffnung erinnert, kann grün blinken. Hinter jedem Feld der Fortschrittsanzeigen 4, 5, 6 sind hierzu entsprechend farbige LEDs installiert.

Eine Steuerungseinheit (nicht dargestellt) im Flüssigkeitsspender 1 ist so programmiert, dass bei Betätigung der Ausgabeeinheit 2 durch die Präsenz einer Hand unterhalb des Handsensors die erste, also die ganz linke, Fortschrittsanzeige 4 im Startpositionsfeld 8 rot zu blinken beginnt. In einer nicht dargestellten Speichereinheit des Flüssigkeitsspenders 1 sind dabei Daten zu einer empfohlenen Gesamtdauer einer Körperreinigungsprozedur hinterlegt sowie Daten, in welcher Geschwindigkeit das visuelle Blinksignal vom Startpositionsfeld 4 über die zwischenliegenden Anzeigefelder zum Endpositionsfeld 7 zu laufen hat, und zu welchem Zeitpunkt das visuelle Blinksignal hierbei von einem Anzeigefeld in das benachbarte Anzeigefeld übergeht und somit auch zu welchem Zeitpunkt das visuelle Blinksignal von einem Farbfeldbereich in einen anderen Farbfeldbereich übergeht, also zu welchen Zeitpunkten das intermittierend translatorisch wandernde Blinksignal jeweils die Farbe wechselt.

Das visuelle Signal der optischen Anzeigeeinheit 3 verweilt also je nach hinterlegten Zeitperioden für definierte Zeiten in einem Anzeigefeld, bevor es in das benachbarte Anzeigefeld wechselt.

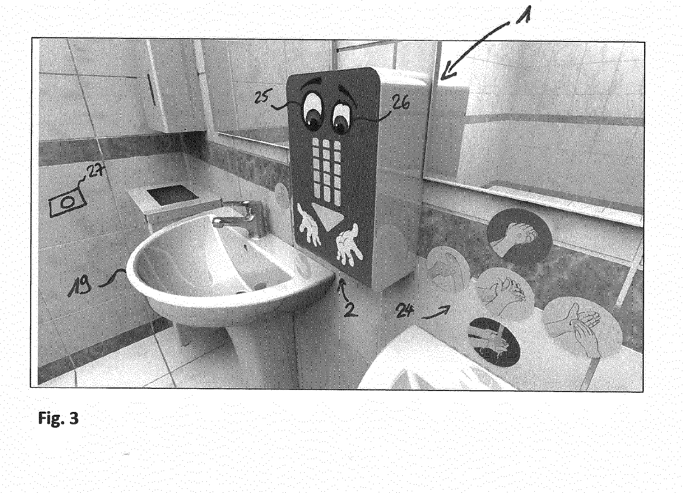

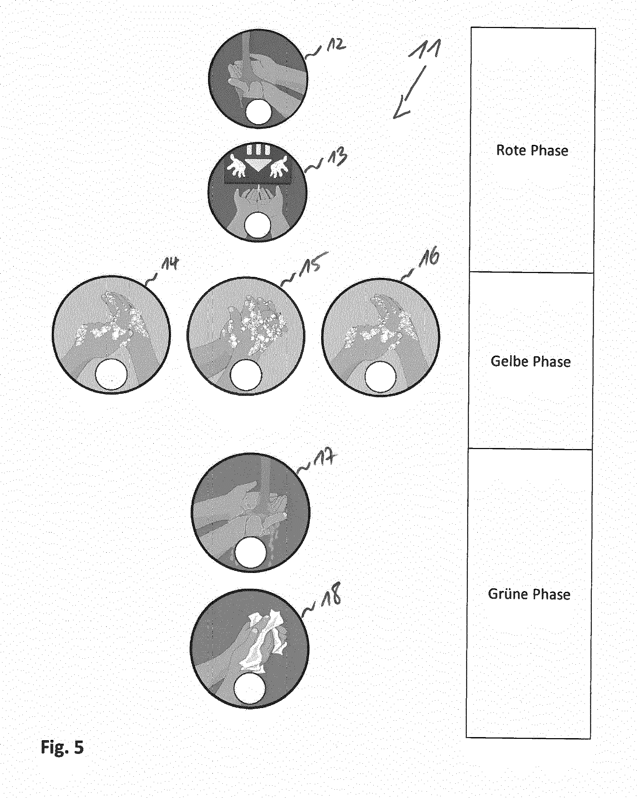

Wie Figur 5 in Zusammenschau mit Figur 3 zu entnehmen ist, umfasst ein erfindungsgemäßes Körperreinigungssystem einen erfindungsgemäßen Flüssigkeitsspender 1 sowie eine visuelle Informationsquelle, hier in der Form einer Handreinigungsanleitung 11, welche hier durch mehrere, teils farblich unterschiedliche Schilder 12-18, genau wie der Flüssigkeitsspender 1 selbst, im Sichtfeld des Bedieners in unmittelbarer Nähe des Waschortes, hier des Waschbeckens 19, installierbar ist. In der Ausführung der Figur 3, welche eine weitere Ausführung einer Handreinigungsanleitung 24 zeigt, sind es insgesamt fünf Schilder (oben ein rotes, in der Mitte drei gelbe und unten ein grünes Schild) und in der Ausführung der Figur 5 sind es insgesamt sieben Schilder (oben zwei rote 12-13, in der Mitte drei gelbe 14-16, und unten zwei grüne Schilder 17-18).

Wie den in Figur 5 dargestellten Schildern 12-18 gut zu entnehmen ist, ordnen die Schilder 12-18 thematisch zusammengehörige Handlungsschritte einer Körperreinigungsprozedur, hier des Händewaschens, über Farbcodes zugehörigen übergeordneten Reinigungsphasen (rot, gelb, grün) zu. Diese Farbcodes werden in den Signalen der Fortschrittsanzeigen 4-6 des Flüssigkeitsspenders 1 entsprechend wiedergegeben, so dass ein Bediener beispielsweise zuordnet kann, wann und wie lange er sich die Hände befeuchten und Seife aufnehmen soll (rote Phase in Figur 5), wann und wie lange er die Hände einseifen soll (gelbe Phase in Figur 5), und wann und wie lange er die Hände abwaschen und trocknen soll (grüne Phase in Figur 5). In Figur 5 sind die zugeordneten Phasen zum leichteren Verständnis nochmals in Form einer Tabelle rechts neben der Handreinigungsanleitung 11 genannt. Die Anzahl der Schilder 12-18 sowie deren Informationsgehalt kann je nach gewünschter Ausführung variieren, so lange die optische Anzeigeeinheit 3 entsprechende optische Merkmale der Informationsquelle 11 wiedergeben kann.

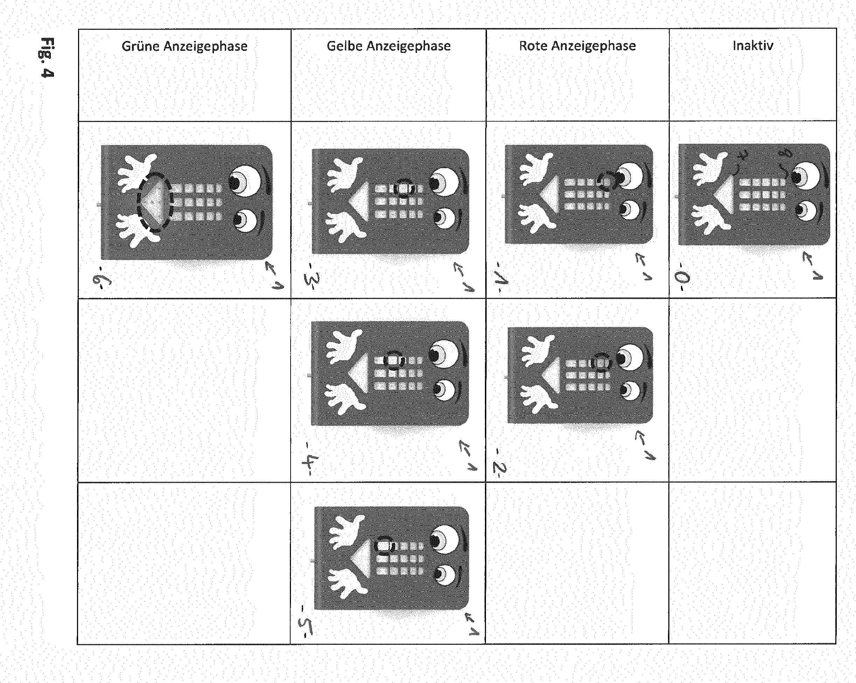

Der zeitliche Ablauf einer empfohlenen Körperreinigungsprozedur und somit die Anleitung eines Bedieners werden nochmals aus Figur 4 gut ersichtlich.

Hierbei wandert das visuelle Lichtsignal (zum leichteren Verständnis jeweils mit einem gestrichelten Kreis umrahmt) intermittierend translatorisch in der Fortschrittsanzeige 4 von dem Startpositionsfeld 8 über einzelne Zwischenfelder zusammen mit einem Farbumschlag von Rot über Gelb zu Grün, hin zum dreieckigen Endpositionsfeld 7. Hierbei ist zur selben Zeit jeweils nur ein Anzeigefeld der Fortschrittsanzeige aktiv. Die einzelnen Zustände sind in Figur 4 von 0 bis 6 durchnummeriert.

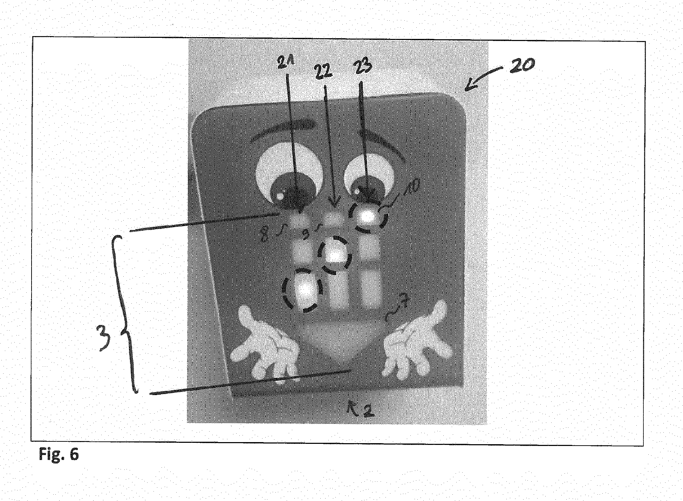

Figur 6 zeigt eine leicht abweichende Ausführungsform eines erfindungsgemäßen Flüssigkeitsspenders 20 mit Fortschrittsanzeigen 21, 22, 23, bei denen jedoch die Anzeigefelder anders aufgeteilt sind und die Anzahl der LEDs hinter den jeweiligen Anzeigefeldern anders als in Fig. 1 angeordnet ist. Die Fortschrittsanzeigen 21, 22, 23 haben dieselbe Funktionsweise wie die Fortschrittsanzeigen 4, 5, 6. In dieser Ausführung blinkt bei einem Durchlauf lediglich das Startpositionsfeld rot, gefolgt von gelben Anzeigefeldern, gefolgt vom grünen dreieckigen Endpositionsfeld 7. Auch hier ist bei einer Reinigungsprozedur pro Fortschrittsanzeige 21, 22, 23 nur ein Anzeigefeld aktiv.

Wie der Figur 6 zu entnehmen ist, können die drei Fortschrittsanzeigen 21, 22, 23, wie auch die Fortschrittsanzeigen 4-6, simultan visuelle Signale für drei gleichzeitig ablaufende, jedoch in ihrem Fortschritt zeitlich versetzte, Körperreinigungsprozeduren anzeigen.

Der Flüssigkeitsspender 1, 20 ist hierbei so programmiert, dass bei einer Betätigung der Ausgabeeinheit 2 über den Handdetektor nach einer gewissen Inaktivitätszeitspanne des Handdetektors, beispielsweise nach 3 Sekunden, automatisch eine weitere Fortschrittsanzeige 5, 22, und bei einer nochmaligen Betätigung nach besagter Inaktivitätszeitspanne, automatisch die dritte Fortschrittsanzeige 6, 23 zu laufen beginnt. Nach einer nochmaligen Betätigung der Ausgabeeinheit nach besagter Inaktivitätszeitspanne ist zwischenzeitlich die erste Fortschrittsanzeige 4, 21 vollständig durchgelaufen und wieder bereit, die neue Reinigungsprozedur anzuzeigen.

Da die einzelnen Fortschrittsanzeigen 4-6, 21-23 durch die Inaktivitätszeitspanne nur zeitlich versetzt laufen können, gibt es keine zeitlichen Überschneidungen im gemeinsamen Endpositionsfeld 7, welches somit für jede Fortschrittsanzeige 4-6, 21-23 nutzbar ist.

Die Flüssigkeitsspender 1, 20 der zuvor genannten Ausführungen weisen weiterhin im oberen, hinteren Bereich ein Funkmodul (nicht abgebildet) auf, welches ein vollständiges Durchlaufen einer Fortschrittsanzeige 4-6, 21-23 an einen externen Empfänger, beispielsweise eine externe Datenbank oder ein entferntes Durchgangsöffnungs- und Schließsystem senden kann.

So kann in einer Ausführungsform ein Durchgang in einen hygienesensiblen Bereich erst durch das Durchgangsöffnungs- und Schließsystem freigegeben werden, wenn zuvor beim Durchgangsöffnungs- und Schließsystem eine Meldung über eine vollständig durchlaufene Körperreinigungsprozedur eingegangen ist.

In einer weiteren, in Figur 3 abgebildeten Ausführungsform ist bei den zuvor genannten Flüssigkeitsspender 1 etwa an der Position der Augen der Spenderfrontseite ein zusätzlicher berührungsloser Bewegungsmelder 26 und eine Kamera 25 als Teil einer im Flüssigkeitsspender integrierten Überwachungssensorik angebracht.

Der zusätzliche berührungslose Bewegungsmelder 26 ist hierbei ein Infrarot-Bewegungsmelder und in den Waschraum gerichtet. Die Kamera 25 ist hierbei auf den Waschbereich selbst, beispielsweise das Waschbecken, gerichtet. Die hierbei gewonnenen Daten von den Bewegungen und Aktionen des Bedieners werden von der Überwachungssensorik mit im Flüssigkeitsspender hinterlegten Daten (Körperbewegungs- und Positionsmuster) verglichen, entsprechend kategorisiert und bewertet. Die optische Anzeigeeinheit 3 zeigt die visuellen Signale in Abhängigkeit von der Bewertung der Bedieneraktionen an und kann beispielsweise den Bediener anleiten, sich länger die Hände einzuseifen oder bei Inaktivität des Bedieners oder bei einem vorzeitigem Verlassen des Waschbereichs die Signalanzeige anhalten oder abbrechen.

In einer weiteren Ausführungsform kann der zuvor genannte Flüssigkeitsspender zusammen mit einem oder mehreren externen berührungslosen Bewegungsmeldern 27, beispielsweise infrarotbasierenden oder schallbasierenden Bewegungsmeldern, ein Körperreinigungssystem bilden, wobei die Daten des mindestens einen externen Bewegungsmelders analysiert und bewertet werden, und in einer möglichen Ausführungsform mit in die zuvor genannte Datenanalyse durch die Überwachungssensorik einfliesen.

Durch den einen oder die mehreren den Waschraum überwachenden Bewegungsmelder, beispielsweise durch einen Bewegungsmelder 26 im Flüssigkeitsspender und mindestens einen externen Bewegungsmelder 27 im Waschraum, können über bekannte Tracking-Methoden, beispielsweise über laufzeitbasierende Berechnungsmethoden und logikbasierende Berechnungsmethoden, die Bewegungen und Handlungen eines Bedieners erfasst und bewertet werden, und die visuelle Signalanzeige der optischen Anzeigeeinheit 3 in bereits beschriebener Weise beeinflussen.

Die Funktionsweise eines möglichen Flüssigkeitsspenders 1, 20 gemäß der vorliegenden Erfindung wird nachfolgend kurz erläutert.

Ein Bediener betritt durch eine Waschraumtür einen Waschraum in dem der Flüssigkeitsspender 1, 20 in unmittelbarere Nähe eines Waschbeckens 19 installiert ist. Zudem ist eine Handwaschanleitung 11, 24 mit Farbcodes im Sichtfeld des Bedieners oberhalb des Waschbeckens installiert. Ein Bediener hat somit beim Benutzen des Waschbeckens 19 Sicht auf die Frontseite des Flüssigkeitsspenders 1, 20 sowie auf die Handwaschanleitung 11, 24.

Beim Öffnen der Waschraumtür erhält der Flüssigkeitsspender ein Türöffnungssignal von einem Waschraumtürsensor. Die optische Anzeigeeinheit 3 lässt kurz mehrere LEDs aufblicken, um die Aufmerksamkeit des Bedieners auf den Flüssigkeitsspender 1, 20 zu lenken.

Die erste Fortschrittsanzeige 4 setzt das Startpositionsfeld 8 auf blinkend Rot. Der Bediener nähert sich weiter dem Flüssigkeitsspender 1, 20, was in dieser Ausführungsform von dem im Flüssigkeitsspender 1, 20 integrierten Raumbewegungsmelder 25 registriert wird. Mit Blick auf die Handwaschanleitung 11, 24 erkennt der Bediener, dass er sich in einem ersten Schritt die Hände befeuchten muss. Die Kamera 26 registriert das Befeuchten der Hände, und erlaubt der Fortschrittsanzeige 4 auf das nachfolgende rote Anzeigefeld zu schalten. Hierdurch bekommt der Bediener, welcher gleichzeitig die Handwaschanleitung 11, 24 im Blickfeld hat, angezeigt, dass er sich nun die Seife ausgeben lassen soll. Nach erfolgter Betätigung des Handdetektors und erfolgter Seifenausgabe durch die Ausgabeeinheit 2 schaltet die Fortschrittsanzeige 4 auf das nachfolgende Anzeigefeld, welches Gelb ist. Der Handwaschanleitung 11 entnimmt der Bediener nun, dass er sich die Hände einseifen muss. Die Fortschrittsanzeige 4 läuft hierbei für definierte Zeitspannen durch die drei gelben Anzeigefelder. Für den Fall, dass die Kamera 26 ein untypisches Handbewegungsmuster oder der integrierte Bewegungsmelder 25 ein Entfernen des Bedieners erfasst, wird beispielsweise die Einseifzeit verlängert oder der Vorgang abgebrochen. Selbiges gilt für den nachfolgenden grünen Seifenabwaschvorgang und Handtrocknungsvorgang, wobei der Bediener Details hierzu wieder der Handwaschanleitung 11, 24 entnehmen kann. Sollte der Bediener sich aus dem Raum entfernen, so kann dies ebenfalls von dem intergierten Bewegungsmelder 25 sowie beispielsweise einem externen Bewegungsmelder 27 oder einem Waschtürsensor registriert werden und an den Flüssigkeitsspender 1, 20 gemeldet werden. Gleichzeitig kann der externe Bewegungsmelder zusammen mit dem im Flüssigkeitsspender 1, 20 integrierten Bewegungsmelder 25 den Waschraum und die Bewegungen des Bedieners überwachen und mit in den Anzeigeverlauf mit einfließen lassen.

Wenn die Handwaschprozedur erfolgreich durchlaufen wurde, blinkt nochmals die Endpositionsanzeige 7 auf, um den Bediener ein positives Feedback zu geben, und der Flüssigkeitsspender 1, 20 sendet dies an eine externe Datenbank, damit die Bedienungsdaten bei Bedarf ausgewertet werden können. Weiterhin sendet der Flüssigkeitsspender 1, 20 ein positives Signal zu einem externen Durchgangsöffnungs- und Schließsystem, welches daraufhin dem Bediener den Zugang zu einem hygienesensitiven Bereich, wie beispielsweise einen Operationssaal, gewährt.

Sollte während des Waschvorgangs vom Flüssigkeitsspender 1, 20 ein zweiter oder dritter Bediener durch zuvor genannte Detektoren erkannt werden, so werden parallel auf der zweiten und dritten Fortschrittsanzeige 5, 22; 6, 23 entsprechende visuelle Signale in der bereits beschriebenen Weise für die jeweiligen weiteren Bediener angezeigt, und nachgeschaltete Aktionen wie bereits beschrieben beeinflusst.

## Patentansprüche

1. Flüssigkeitsspender (1, 20) zum Anleiten einer Körperreinigungsprozedur, insbesondere einer Handreinigungsprozedur, aufweisend
eine betätigbare Ausgabeeinheit (2) für Hygieneflüssigkeit, und
eine optische Anzeigeeinheit (3), welche mit der Ausgabeeinheit (2) in Verbindung steht,
wobei der Flüssigkeitsspender (1, 20) dazu eingerichtet ist, dass die optische Anzeigeeinheit (3), nach einer Betätigung der Ausgabeeinheit (2), einem Bediener visuelle Signale über eine hinterlegte Zeitdauer einer empfohlenen Körperreinigungsprozedur anzeigt, **dadurch gekennzeichnet, dass**
die optische Anzeigeeinheit (3) dazu eingerichtet ist, gleichzeitig unterschiedliche visuelle Signale zu parallel ablaufenden Körperreinigungsprozeduren mehrerer Bediener anzuzeigen,
und die optische Anzeigeeinheit (3) mehrere (4-6, 21-23) optische Fortschrittsanzeigen für Körperreinigungsprozeduren in Form von mehreren nebeneinanderliegenden optischen Anzeigebahnen aufweist.

2. Flüssigkeitsspender (1, 20) nach dem vorherigen Anspruch, **dadurch gekennzeichnet, dass**
der Flüssigkeitsspender (1, 20) dazu eingerichtet ist, dass sich die visuellen Signale entsprechend einer hinterlegten zeitlichen Abfolge derart verändern, dass es einem Bediener möglich ist, diese Veränderungen unterschiedlichen Handlungsschritten einer empfohlenen Körperreinigungsprozedur zuzuordnen.

3. Flüssigkeitsspender (1, 20) nach einem der vorherigen Ansprüche, **dadurch gekennzeichnet, dass**
die optischen Anzeigebahnen sektionierte Lichtleisten aufweisen.

4. Flüssigkeitsspender (1, 20) nach einem der vorherigen Ansprüche, **dadurch gekennzeichnet, dass**
die optische Fortschrittsanzeigen (4-6; 21-23) jeweils dazu eingerichtet sind, die visuellen Signale von einem Startpositionsfeld (8-10) der optischen Fortschrittsanzeige hin zu einem Endpositionsfeld (7) der optischen Fortschrittsanzeige laufen zu lassen, insbesondere intermittierend und/oder translatorisch laufen zu lassen.

5. Flüssigkeitsspender (1, 20) nach einem der vorherigen Ansprüche, **dadurch gekennzeichnet, dass**
die optische Anzeigeeinheit (3), insbesondere die optische Fortschrittsanzeigen (4-6; 21-23), dazu eingerichtet ist, die visuellen Signale positionsabhängig inhaltlich zu verändern, insbesondere positionsabhängig in unterschiedlichen Farben anzuzeigen.

6. Flüssigkeitsspender (1, 20) nach einem der vorherigen Ansprüche, **dadurch gekennzeichnet, dass**
die optische Fortschrittsanzeigen (4-6; 21-23) jeweils an ihrem Endpositionsfeld (7) ein im Vergleich zu den vorangegangenen Positionen der optischen Fortschrittsanzeige visuell hervorgehobenes Signal ausgeben können, und/oder mehrere optische Fortschrittsanzeigen (4-6; 21-23) ein gemeinsames Endpositionsfeld (7) aufweisen.

7. Flüssigkeitsspender (1, 20) nach einem der vorherigen Ansprüche, **dadurch gekennzeichnet, dass** der Flüssigkeitsspender (1, 20) einen Empfänger, insbesondere einen Funkempfänger, aufweist, welcher ein externes Türöffnungssignal empfangen kann, und der Flüssigkeitsspender dazu eingerichtet ist, bei einem Empfang eines Türöffnungssignals mindestens ein visuelles Signal auf der optischen Anzeigeeinheit (3) anzuzeigen.

8. Flüssigkeitsspender (1, 20) nach einem der vorherigen Ansprüche, **dadurch gekennzeichnet, dass**
der Flüssigkeitsspender (1, 20) eine berührungslose Überwachungssensorik (25, 26) aufweist, welche mit der optischen Anzeigeeinheit (3) verbunden ist und welche dazu eingerichtet ist, während einer Körperreinigungsprozedur die Bewegungen und/oder Positionen eines Bedieners zu detektieren und basierend auf hinterlegten Bewegungs- und/oder Positionsmustern zu bewerten, und die optische Anzeigeeinheit (3) dazu eingerichtet ist, die visuellen Signale bei einer Körperreinigungsprozedur in Abhängigkeit von der Bewertung der Überwachungssensorik anzuzeigen.

9. Flüssigkeitsspender (1, 20) nach einem der vorherigen Ansprüche, **dadurch gekennzeichnet, dass**
der Flüssigkeitsspender einen Sender, insbesondere einen Funksender, aufweist, welcher ein Signal an einen externen Empfänger, insbesondere an ein Durchgangsöffnungs- und Schließsystem, senden kann, und der Flüssigkeitsspender dazu eingerichtet ist, ein erfolgtes vollständiges Durchlaufen einer Körperreinigungsprozedur an den externen Empfänger, insbesondere an das Durchgangsöffnungs- und Schließsystem, zu melden.

10. Körperreinigungssystem, insbesondere Handreinigungssystem,
**gekennzeichnet durch**
einen Flüssigkeitsspender (1, 20) nach einem der vorherigen Ansprüche, insbesondere nach Anspruch 8, und durch
mindestens einen externen berührungslosen Bewegungsmelder, welcher im Umfeld des Flüssigkeitsspenders installierbar ist und mit dem Flüssigkeitsspender kommunizieren kann, und der Flüssigkeitsspender, insbesondere die Überwachungssensorik, dazu eingerichtet ist, aus den gewonnenen Bewegungs- und/oder Positionsdaten Rückschlüsse auf die Aktionen des Bedieners zu ziehen und diese zu bewerten, und die optische Anzeigeeinheit dazu eingerichtet ist, die visuellen Signale bei einer Körperreinigungsprozedur in Abhängigkeit von der Bewertung anzuzeigen.

11. Körperreinigungssystem, insbesondere Handreinigungssystem, **gekennzeichnet durch**
einen Flüssigkeitsspender (1, 20) gemäß einem der vorherigen Ansprüche 1 bis 9, und
eine visuelle Informationsquelle (11, 24), insbesondere eine Körperreinigungsanleitung, beispielsweise eine Handreinigungsanleitung, welche unterschiedliche optische Merkmale, insbesondere unterschiedliche Farbkennzeichnungen, entsprechend einzelner Phasen einer Körperreinigungsprozedur enthält, und
die optische Anzeigeeinheit des Flüssigkeitsspenders derart eingerichtet ist, dass die angezeigten visuellen Signale zu einer Körperreinigungsprozedur, je nach Zeitfortschritt, besagte phasenzugehörige optische Merkmale, insbesondere die entsprechenden Farbkennzeichnungen, beinhalten.

12. Verfahren zum Anleiten einer Körperreinigungsprozedur, insbesondere einer Handreinigungsprozedur, umfassend
ein Detektieren einer Betätigung einer Ausgabeeinheit (2) eines Hygieneflüssigkeitsspenders (1, 20) durch einen Bediener,
**gekennzeichnet durch**
ein Anzeigen von visuellen Signalen über eine hinterlegte Zeitdauer einer empfohlenen Körperreinigungsprozedur auf einer optischen Anzeigeeinheit (3), und **dadurch, dass** bei einer Betätigung der Ausgabeeinheit (2) während einer bereits laufenden Signalanzeige einer Körperreinigungsprozedur auf einer Fortschrittsanzeige in Form einer Anzeigebahn der Anzeigeeinheit (3), gleichzeitig visuelle Signale für eine weitere Körperreinigungsprozedur eines weiteren Bedieners auf einer weiteren Fortschrittsanzeige in Form einer weiteren Anzeigebahn der optischen Anzeigeeinheit (3) angezeigt werden.

13. Verfahren nach dem vorherigen Anspruch, **dadurch gekennzeichnet, dass**
sich beim Anzeigen die visuellen Signale entsprechend einer hinterlegten zeitlichen Abfolge derart verändern, dass es einem Bediener möglich ist, diese Veränderungen unterschiedlichen Handlungsschritten einer empfohlenen Körperreinigungsprozedur zuzuordnen.

14. Verfahren nach Anspruch 12 oder 13, **dadurch gekennzeichnet, dass**
dem Bediener während dem Anzeigen der visuellen Signale, zusätzliche unterschiedliche optische Merkmale, insbesondere unterschiedliche Farbkennzeichnungen, präsentiert werden, welche einzelnen Phasen einer empfohlenen Körperreinigungsprozedur entsprechen, und
die visuellen Signale der optischen Anzeigeeinheit (3), je nach Zeitfortschritt, besagte phasenzugehörige optische Merkmale, insbesondere die entsprechenden Farbkennzeichnungen, enthalten.

## Claims

1. Liquid dispenser device (1, 20) for guiding through a body cleansing procedure, in particular a hand washing procedure, including
an operable dispensing unit (2) for hygienic liquid, and
an optical display unit (3) communicating with the output unit (2),
wherein the liquid dispenser device (1, 20) is arranged such that the optical display unit (3), upon actuation of the dispensing unit (2), displays visual signals to an operator about a stored time duration of a recommended body cleansing procedure,
**characterized in that**
the optical display unit (3) is arranged to simultaneously display different visual signals concerning concurrent body cleansing procedures of several operators,
and the optical display unit (3) comprises a plurality of optical progress indicators (4-6, 21-23) for body cleansing procedures in the form of a plurality of juxtaposed optical display bars.

2. Liquid dispenser device (1, 20) according to the preceding claim,
**characterized in that**
the liquid dispenser device (1, 20) is arranged such that the visual signals change according to a stored time sequence so that an operator is enabled to assign these changes to different steps of a recommended body cleansing procedure.

3. Liquid dispenser device (1, 20) according to any one of the preceding claims, **characterized in that**
the optical display bars include sectionalized light strips.

4. Liquid dispenser device (1, 20) according to any one of the preceding claims, **characterized in that**
the optical progress indicators (4-6; 21-23) are each arranged to cause the visual signals to proceed from a start position field (8-10) of the optical progress indicator to an end position field (7) of the optical progress indicator, in particular to proceed intermittently and/or translationally.

5. Liquid dispenser device (1, 20) according to any one of the preceding claims, **characterized in that**
the optical display unit (3), in particular the optical progress indicators (4-6; 21-23) is arranged to change the content of the visual signals depending on the position, in particular to display them in different colours depending on the position.

6. Liquid dispenser device (1, 20) according to any one of the preceding claims, **characterized in that**
the optical progress indicators (4-6; 21-23) can each output at their end position field (7) a signal visually highlighted in comparison with the previous positions of the optical progress indicator, and/or a plurality of optical progress indicators (4-6; 21-23) share one end position field (7).

7. Liquid dispenser device (1, 20) according to any one of the preceding claims, **characterized in that** the liquid dispenser (1, 20) includes a receiver, in particular a radio receiver able to receive an external door opening signal, and the liquid dispenser device is arranged to display at least a visual signal on the optical display unit (3) upon receipt of a door opening signal.

8. Liquid dispenser device (1, 20) according to any one of the preceding claims, **characterized in that**
the liquid dispenser device (1, 20) includes a contactless monitoring sensor means (25, 26) connected to the optical display unit (3) and arranged to detect the movements and/or positions of an operator during a body cleansing procedure and to evaluate them on the basis of stored movement and/or position patterns, and the optical display unit (3) is arranged to display the visual signals during a body cleansing procedure as a function of the evaluation of the monitoring sensor means.

9. Liquid dispenser device (1, 20) according to any one of the preceding claims, **characterized in that**
the liquid dispenser device includes a transmitter, in particular a radio transmitter able to transmit a signal to an external receiver, in particular to a passage opening and closing system, and the liquid dispenser device is arranged to report a completed passing through a body cleansing procedure to the external receiver, in particular to the passage opening and closing system.

10. Body cleansing system, in particular a hand washing system,
**characterized by**
a liquid dispenser device (1, 20) according to any one of the preceding claims, in particular according to claim 8, and by
at least one external contactless motion sensor which can be installed in the vicinity of the liquid dispenser device and can communicate with the liquid dispenser device, and the liquid dispenser device, in particular the monitoring sensor means, is arranged to draw conclusions about the actions of the operator from the obtained motion and/or position data and to evaluate the same, and the optical display unit is arranged to display the visual signals during a body cleansing procedure depending on the evaluation.

11. Body cleaning system, in particular a hand washing system,
**characterized by**
a liquid dispenser device (1, 20) according to any one of the preceding claims 1 to 9, and
a source (11, 24) of visual information, in particular a body cleansing guide, for example a hand washing guide, which includes different optical features, in particular different color codes corresponding to individual phases of a body cleansing procedure, and
wherein the optical display unit of the liquid dispenser is arranged such that the displayed visual signals for a body cleansing procedure include, depending on the progress in time, said optical features associated with the phases, in particular the respective color codes.

12. Method for guiding through a body cleansing procedure, in particular a hand washing procedure, comprising
detecting an operation of a dispensing unit (2) of a dispenser device (1, 20) for hygienic liquid by an operator,
**characterized by**
displaying visual signals for the duration of a stored period of time of a recommended body cleansing procedure on an optical display unit (3), and in that
when the output unit (2) is operated while a signal of a body cleansing procedure is already being displayed on a progress indicator in the form of a display bar of the display unit (3), visual signals for another body cleansing procedure of another operator are simultaneously displayed on another progress display in the form of an additional display bar of the optical display unit (3).

13. The method according to the preceding claim,
**characterized in that**
the visual signals, while being displayed, change according to a stored time sequence so that an operator is enabled to assign these changes to different steps of a recommended body cleansing procedure.

14. Method according to claims 12 or 13,
**characterized in that**
while displaying the visual signals additional different optical features, in particular different color codes, which correspond to individual phases of a recommended body cleansing procedure are presented to the operator, and
the visual signals of the optical display unit (3) include, depending on the progress in time, said phase-related optical features, in particular the corresponding color codes.

## Revendications

1. Distributeur de liquide (1, 20) pour guider une procédure de nettoyage du corps, en particulier une procédure de nettoyage des mains, comprenant
une unité de distribution actionnable (2) pour le liquide d'hygiène, et
une unité d'affichage optique (3) qui est en communication avec l'unité de distribution (2),
le distributeur de liquide (1, 20) étant adapté pour que l'unité d'affichage optique (3), après un actionnement de l'unité de distribution (2), affiche à un opérateur des signaux visuels concernant une durée mémorisée d'une procédure de nettoyage corporel recommandée, **caractérisé en ce que**
l'unité d'affichage optique (3) est adaptée pour afficher simultanément des signaux visuels différents concernant des procédures de nettoyage corporel de plusieurs opérateurs se déroulant en parallèle,
et l'unité d'affichage optique (3) comprenant une pluralité (4-6, 21-23) d'affichages optiques de progression pour des procédures de nettoyage du corps sous la forme d'une pluralité de voies d'affichage optiques juxtaposées.

2. Distributeur de liquide (1, 20) selon la revendication précédente, **caractérisé en ce que**
le distributeur de liquide (1, 20) est conçu de telle sorte que les signaux visuels se modifient selon une séquence temporelle enregistrée de telle sorte qu'il est possible à un opérateur d'associer ces modifications à différentes étapes d'action d'une procédure de nettoyage corporel recommandée.

3. Distributeur de liquide (1, 20) selon l'une des revendications précédentes, **caractérisé en ce que**
les chemins d'affichage optiques présentent des barres lumineuses en sections.

4. Distributeur de liquide (1, 20) selon l'une des revendications précédentes, **caractérisé en ce que**
les indicateurs optiques de progression (4-6 ; 21-23) sont chacun adaptés pour faire défiler, en particulier de manière intermittente et/ou par translation, les signaux visuels d'un champ de position de départ (8-10) de l'indicateur optique de progression vers un champ de position finale (7) de l'indicateur optique de progression.

5. Distributeur de liquide (1, 20) selon l'une des revendications précédentes, **caractérisé en ce que**
l'unité d'affichage optique (3), en particulier les affichages optiques de progression (4-6 ; 21-23), est conçue pour modifier le contenu des signaux visuels en fonction de la position, en particulier pour les afficher dans différentes couleurs en fonction de la position.

6. Distributeur de liquide (1, 20) selon l'une quelconque des revendications précédentes, **caractérisé en ce que**
les affichages optiques de progression (4-6 ; 21-23) peuvent émettre chacun sur leur champ de position finale (7) un signal visuellement mis en évidence par rapport aux positions précédentes de l'affichage optique de progression, et/ou plusieurs affichages optiques de progression (4-6 ; 21-23) présentent un champ de position finale (7) commun.

7. Distributeur de liquide (1, 20) selon l'une des revendications précédentes, **caractérisé en ce que** le distributeur de liquide (1, 20) comprend un récepteur, en particulier un récepteur radio, qui peut recevoir un signal d'ouverture de porte externe, et le distributeur de liquide est adapté pour afficher au moins un signal visuel sur l'unité d'affichage optique (3) lors de la réception d'un signal d'ouverture de porte.

8. Distributeur de liquide (1, 20) selon l'une quelconque des revendications précédentes, **caractérisé en ce que**
le distributeur de liquide (1, 20) présente un système de capteurs de surveillance sans contact (25, 26) qui est relié à l'unité d'affichage optique (3) et qui est conçu pour détecter les mouvements et/ou les positions d'un opérateur pendant une procédure de nettoyage corporel et pour les évaluer sur la base de modèles de mouvements et/ou de positions enregistrés, et l'unité d'affichage optique (3) est conçue pour afficher les signaux visuels lors d'une procédure de nettoyage corporel en fonction de l'évaluation du système de capteurs de surveillance.

9. Distributeur de liquide (1, 20) selon l'une des revendications précédentes, **caractérisé en ce que**
le distributeur de liquide comprend un émetteur, en particulier un émetteur radio, qui peut envoyer un signal à un récepteur externe, en particulier à un système d'ouverture et de fermeture de passage, et le distributeur de liquide est conçu pour signaler au récepteur externe, en particulier au système d'ouverture et de fermeture de passage, qu'une procédure de nettoyage corporel a été entièrement effectuée.

10. Système de nettoyage du corps, en particulier des mains,
**caractérisé par**
un distributeur de liquide (1, 20) selon l'une quelconque des revendications précédentes, en particulier selon la revendication 8, et par
au moins un détecteur de mouvement externe sans contact, qui peut être installé dans l'environnement du distributeur de liquide et qui peut communiquer avec le distributeur de liquide, et le distributeur de liquide, en particulier le système de capteurs de surveillance, est conçu pour tirer des conclusions sur les actions de l'opérateur à partir des données de mouvement et/ou de position obtenues et pour les évaluer, et l'unité d'affichage optique est conçue pour afficher les signaux visuels lors d'une procédure de nettoyage du corps en fonction de l'évaluation.

11. Système de nettoyage du corps, en particulier système de nettoyage des mains, **caractérisé par**
un distributeur de liquide (1, 20) selon l'une quelconque des revendications 1 à 9 précédentes, et
une source d'informations visuelles (11, 24), en particulier un guide de nettoyage corporel, par exemple un guide de nettoyage des mains, qui contient différentes caractéristiques visuelles, en particulier différents codes de couleur, correspondant à des phases individuelles d'une procédure de nettoyage corporel, et
l'unité d'affichage optique du distributeur de liquide est agencée de telle sorte que les signaux visuels affichés pour une procédure de nettoyage corporel contiennent, en fonction de l'avancement dans le temps, lesdites caractéristiques optiques associées aux phases, en particulier les codes de couleur correspondants.

12. Méthode pour guider une procédure de nettoyage du corps, en particulier une procédure de nettoyage des mains, comprenant
une détection d'un actionnement d'une unité de distribution (2) d'un distributeur de liquide d'hygiène (1, 20) par un opérateur,
**caractérisé par**
un affichage sur une unité d'affichage optique (3) de signaux visuels concernant une durée mémorisée d'une procédure de nettoyage corporel recommandée, et en ce que
lors d'un actionnement de l'unité de distribution (2) pendant un affichage de signaux déjà en cours d'une procédure de nettoyage corporel sur un affichage de progression sous la forme d'une voie d'affichage de l'unité d'affichage optique (3), des signaux visuels pour une autre procédure de nettoyage corporel d'un autre opérateur sont affichés simultanément sur un autre affichage de progression sous la forme d'une autre voie d'affichage de l'unité d'affichage optique (3).

13. Procédé selon la revendication précédente, **caractérisé en ce que**
lors de l'affichage, les signaux visuels se modifient selon une séquence temporelle mémorisée de telle sorte qu'il est possible à un opérateur d'associer ces modifications à différentes étapes d'action d'une procédure de nettoyage corporel recommandée.

14. Procédé selon la revendication 12 ou 13, **caractérisé en ce que**
pendant l'affichage des signaux visuels, l'opérateur se voit présenter des caractéristiques optiques supplémentaires différentes, en particulier des codes de couleur différents, qui correspondent à des phases individuelles d'une procédure de nettoyage corporel recommandée, et
les signaux visuels de l'unité d'affichage optique (3) contiennent, en fonction de l'avancement dans le temps, lesdites caractéristiques optiques associées à la phase, en particulier les caractéristiques de couleur correspondantes.
